# EUROPÄISCHE PATENTSCHRIFT

(11) **EP 1 121 088 B1**
(45) Veröffentlichungstag und Bekanntmachung des Hinweises auf die Patenterteilung: **18.12.2002**
(21) Anmeldenummer: 99950616.5
(22) Anmeldetag: 05.10.1999
(51) Int. Cl.: A61K 7/00, A61K 7/06, A61K 7/48, A61K 9/51

(54) **VERWENDUNG VON NANOSKALIGEN STEROLEN UND STEROLESTERN**
USE OF NANOSCALE STEROLS AND STEROL ESTERS
UTILISATION DE STEROLS ET DE STEROLS ESTERIFIES DE L'ORDRE DU NANOMETRE

(30) Priorität: 14.10.1998 US 104144 P
(43) Veröffentlichungstag der Anmeldung: 08.08.2001
(73) Patentinhaber: Cognis Deutschland GmbH & Co. KG, 40589 Düsseldorf (DE)
(72) Erfinder: FÖRSTER, Thomas, D-40699 Erkrath (DE); FABRY, Bernd, D-41352 Korschenbroich (DE); HOLLENBROCK, Martina, D-40723 Hilden (DE); KROPF, Christian, D-40597 Düsseldorf (DE)
(86) Internationale Anmeldenummer: EP9907359
(87) Internationale Veröffentlichungsnummer: WO00021490

(56) Entgegenhaltungen:
- EP-A- 0 506 197
- WO-A-92/21331
- WO-A-96/14830
- FR-A- 2 642 305
- US-A- 4 508 703
- US-A- 5 445 811
- US-A- 5 576 016
- "Micronization of organic solids by rapid expansion of supercritical solutions" STN INTERNATIONAL, FILE CHEM. ABS., AN=129:277888, XP002126942 & WORLD CONGR. PART. TECHNOL. 3 (1998), 4037-4047 PUBLISHER: INSTITUTION OF CHEMICAL ENGINEERS, RUGBY UK. CODEN: 66PSA9,
- "Effect of process parameters on particles obtained by rapid expansion of supercritical solutions" STN INTERNATIONAL, FILE CHEM. ABS., AN=129:278061, XP002126943 & WORLD CONGR. PART. TECHNOL. 3 (1998), 4047-4057 PUBLISHER: INSTITUTION OF CHEMICAL ENGINEERS, RUGBY UK. CODEN: 66PSA9,

## Beschreibung

### Gebiet der Erfindung

Die Erfindung befindet sich auf dem Gebiet der Nanopartikel und betrifft die Verwendung von nanoskaligen Sterolen und Sterolestern in der Kosmetik.

### Stand der Technik

Sterole und Sterolester stellen wichtige Rohstoffe sowohl für die Kosmetik und Pharmazie als auch für die Nahrungsmittelindustrie dar. So gibt es beispielsweise Hinweise dafür, daß Sterole, insbesondere pflanzliche Vertreter ("Phytosterole") in die Basalmembran der Haut eingebaut werden und über die Differenzierung der Hautzellen an die Hautoberfläche gelangen. Dies würde die pflegende und schützende Wirkung von Phytosterolen in der Hautkosmetik erklären. Die topische Anwendung von Sterolen führt auch zu einer erhöhten Hautfeuchtigkeit und einem gesteigerten Lipidgehalt. Hierdurch wird das Schuppungsverhalten der Haut verbessert und vorhandene Erytheme reduziert. Nach neueren Untersuchungen wirken Sterole (und hier wieder vorzugsweise Phytosterole) über eine Reduzierung des Leucotrienspiegels auf die Arachidonsäure-Kaskade ein. Erhöhte Konzentrationen an Leucotrienen in der Haut sind jedoch immer mit entzündlichen Reaktionen, z.B. bei atopischer Dermatitis, Psoriasis und UV-Erythemen, verbunden; Sterole besitzen daher auch eine entzündungshemmende Wirkung. Im Bereich der Haarpflege zeigen sterolhaltige Zubereitungen eine Verbesserung der Kämmbarkeit und Reißfestigkeit, insbesondere bei blondiertem Haar. Übersichten zu den Eigenschaften von Sterolen und Sterolestern in der Kosmetik finden sich beispielsweise von R.Wachter in **Parf.Kosm. 75, 755 (1994)** bzw. **Cosm.Toil. 110, 72 (1995).** Des weiteren sei auf die Deutsche Patentanmeldung **DE-A1 19522822** (Henkel) verwiesen, in der Mischungen von Sterolen und Fettalkoholen für kosmetische Anwendungen vorgeschlagen werden. Die antimikrobielle Wirkung von Sterolen und Sterolderivaten ist beispielsweise aus der europäischen Patentanmeldung **EP-A1 0838155** (Beiersdorf) bekannt.

Die Wirkung der Sterole und Sterolester hängt stets mit der Geschwindigkeit zusammen, mit der die Verbindungen eingebaut bzw. resorbiert werden. Hier besteht für die verfügbaren Stoffe des Stands der Technik noch ein erhebliches Verbesserungspotential. Die Aufgabe der vorliegenden Erfindung hat somit darin bestanden, die Aufnahme von Sterole und Sterolester bei topischer Applikation durch Bereitstellung neuer Anbietungsformen zu beschleunigen.

### Beschreibung der Erfindung

Gegenstand der Erfindung ist die Verwendung von nanoskaligen Sterolen und/oder Sterolestern mit Teilchendurchmessern im Bereich von 10 bis 300 nm zur Herstellung von kosmetischen und/oder pharmazeutischen Zubereitungen.

Überraschenderweise wurde gefunden, daß sich die Resorption von Sterolen und Sterolestern, insbesondere solcnen auf Basis pflanzlicher Rohstoffe, signifikant steigern läßt, wenn diese in Form von Nanoteilchen, d.h. Partikeln mit einem mittleren Durchmesser im Bereich von 10 bis 300 und vorzugsweise 50 bis 150 nm vorliegen.

### Sterole und Sterolester

Unter Sterolen (oder synonym Stenolen) sind tierische bzw. pflanzliche Steroide zu verstehen, die nur am C-3 eine Hydroxylgruppe, sonst aber keine funktionellen Gruppen tragen. In der Regel besitzen die Sterole 27 bis 30 Kohlenstoffatome und eine Doppelbindung in 5/6, gegebenenfalls 7/8. 8/9 oder anderen Positionen. Neben diesen ungesättigten Spezies kommen als Sterole auch die durch Härtung erhältlichen gesättigten Verbindungen in Frage, die als Stanole bezeichnet werden und von der vorliegenden Erfindung mitumschlossen werden. Ein Beispiel für ein geeignetes tierisches Sterol ist Cholesterol. Typische Beispiele für geeignete Phytosterole, welche aus anwendungstechnischen Gründen bevorzugt werden, sind beispielsweise Ergosterole, Campesterole, Stigmasterole, Brassicasterole sowie vorzugsweise Sitosterole bzw. Sitostanole und insbesondere β-Sitosterole bzw. β-Sitostanole. Neben den genannten Phytosterolen werden vorzugsweise deren Ester eingesetzt. Die Säurekomponente der Esters kann auf Carbonsäuren der Formel **(I)** zurückgehen.

**R**^{**1**}**CO-OH (I)**

in der R¹CO für einen aliphatischen, linearen oder verzweigten Acylrest mit 2 bis 22 Kohlenstoffatomen und 0 und/oder 1, 2 oder 3 Doppelbindungen steht. Typische Beispiele sind Essigsäure, Propionsäure, Buttersäure, Valeriansäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, konjugierte Linolsäure (CLA), Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Besonders bevorzugt ist der Einsatz von estern des β-Sitosterols bzw. β-Sitostanols mit Fettsäuren mit 12 bis 18 Kohlenstoffatomen. Diese Ester können sowohl durch direkte Veresterung der Phytostenole mit den Fettsäuren oder aber durch Umesterung mit Fettsäureniedrigalkylestern oder Triglyceriden in Gegenwart geeigneter Katalysatoren, wie z.B. Natriumethylat oder speziell auch Enzymen hergestellt werden [vgl. **EP-A2 0195311** (Yoshikawa)].

### Herstellung von Nanopartikeln

Ein solches Verfahren zur Herstellung von Nanoteilchen durch rasche Entspannung von überkritischen Lösungen **(Rapid Expansion of Supercritical Solutions RESS)** ist beispielsweise aus dem Aufsatz von S.Chihlar, M.Türk und K.Schaber in **Proceedings World Congress on Particle Technology 3, Brighton, 1998** bekannt. Um zu verhindern, daß die Nanoteilchen wieder zusammenbacken, empfiehlt es sich, die Ausgangsstoffe in Gegenwart geeigneter Schutzkolloide oder Emulgatoren zu lösen und/oder die kritischen Lösungen in wäßrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können. Geeignete Schutzkolloide sind dabei z.B. Gelatine, Casein, Gummi arabicum, Lysalbinsäure, Stärke sowie Polymere, wie etwa Polyvinylalkohole, Polyvinylpyrrolidone Polyalkylenglycole und Polyacrylate. Die bevorzugt zu verwendenden nanoskaligen Sterole und/oder Sterolester sind also die, die von einem Schutzkolloid und/oder einem Emulgator ummantelt vorliegen. Üblicherweise werden die Schutzkolloide oder Emulgatoren in Mengen von 0,1 bis 20, vorzugsweise 5 bis 15 Gew.-% - bezogen auf die Sterole bzw. Sterolester - eingesetzt.

Ein weiteres geeignetes Verfahren zur Herstellung der nanoskaligen Teilchen bietet die **Evaporationstechnik**. Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten **GAS-Verfahren** (Gas Anti Solvent Recrystallization). Das Verfahren nutzt ein hochkomprirniertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das **PCA-Verfahren** (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim **PGSS-Verfahren** (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmeiztemperatur, der Viskosität und der Oberflächenspannung. Bei der Expansion durch eine Düse kommt es durch Abkühlungseffekte zur Bildung feinster Teilchen.

### Gewerbliche Anwendbarkeit

Gegenüber Sterolen und Sterolestern des Stands der Technik bewirkt die besondere Feinteiligkeit der Partikel bei topischer Anwendung ihr rascheres Eindringen in das Stratum Comeum. Die Einsatzmenge der nanoskaligen Verbindungen liegt üblicherweise in der Größenordnung von 0,1 bis 5, vorzugsweise 0,5 bis 3 und insbesondere 1 bis 2 Gew.-% - bezogen auf die Zubereitungen.

### Kosmetische und/oder pharmazeutische Zubereitungen

Die unter erfindungsgemäßer Verwendung der nanoskaligen Sterole und Sterolester erhältlichen Zubereitungen, wie beispielsweise Haarshampoos, Haarlotionen, Schaumbäder, Cremes, Lotionen oder Salben, können ferner als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Polymere, Siliconverbindungen, biogene Wirkstoffe, Deowirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine undloder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkohoien, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Potyolen, Siliconöle und/oder aliphatische bzw. naphthenische Kohlenwasserstoffe in Betracht. Diese Ölkörper können auch schon bei der Herstellung der Nanoteilchen eingesetzt werden und dann als Medium dienen, in welches die fluiden Lösungen entspannt werden.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkyfglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Emulgatoren dienen Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyfsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)-phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Die genannten nichtionischen, anionischen, kationischen oder amphoteren bzw. zwitterionischen Emulgatoren können ebenso wie die Ölkörper als Co-Solventien oder als Medien dienen, in welche die fluiden Mischungen entspannt werden.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen undloder Potyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhenmolekulare Polyethytenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxy-propyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere,Octylacrylamid/Methylmethacrylat/tert.Butylaminoethylmethacrylat/2Hydroxypropylmethacrylat-Copolymere, Polyvinylpyrrolidon, Vinylpyrrolidon/Vinylacetat-Copolymere,Vinylpyrroli-don/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm.Toil. 91,27 (1976).**

Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung (vgl. **J.Soc.Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester,
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester,
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsutfoximine, Homocysteinsuifoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Metnionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-lsomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl. Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Zur Herstellung der nanoskaligen Sterole und Sterolester (**Beispiele 1 bis 5**) wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 I/h auf den gewünschten überkritischen Druck p verdichtet. Anschließend wurde das Lösungsmittel in einem Vorheizer auf die erforderliche Temperatur T1 gebracht und in eine Extraktionskolonne (Stahl, 400 ml) geleitet, welche mit dem Sterol bzw. Sterolester beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.-%ige wäßrige Lösung eines Emulgators bzw. Schutzkolloids enthielt. Das fluide Medium verdampfte und zurück blieben die im Schutzkolloid eingeschlossenen, dispergierten Nanopartikel. Zur Herstellung der Nanoteilchen gemäß **Beispiel 6** wurde eine 1 Gew.-%ige Lösung von Phytosterol in Aceton unter starkem Rühren bei 40°C und einem verminderten Druck von 40 mbar in eine 4 Gew.-% wäßrige Lösung von Coco Glucosides getropft. Das verdampfende Lösungsmittel wurde in einer Kühlfalle kondensiert, während die Dispersion mit den Nanopartikeln zurückblieb. Die Verfahrensbedingungen und der mittlere Partikelgrößenbereich (photometrisch nach der 3-WEM-Methode bestimmt) sind in der nachfolgenden Tabelle 1 angegeben.

**Tabelle 1**

| **Nanopartikel** | | | | | | | |
|---|---|---|---|---|---|---|---|
| **Bsp.** | **Sterol/Sterolester** | **Lsgm.** | **p** **bar** | **T1** **°C** | **T2** **°C** | **Emuigator/Schutzkolioid** | **PGB** **nm** |
| 1 | Phytosterol* | CO₂ | 200 | 80 | 175 | Polyvinylalkohol | 50-125 |
| 2 | Phytosterol* | CO₂ | 180 | 70 | 160 | Polyethylenglycol (M=400) | 70-130 |
| 3 | β-Sitostanol | CO₂ | 200 | 85 | 180 | Polyvinylalkohol | 70-140 |
| 4 | β-Sitostenyllaurat | CO₂ | 200 | 85 | 175 | Polyvinylalkohol | 50-150 |
| 5 | β-Sitostanylstearat | CO₂ | 200 | 85 | 175 | Coco Glucosides | 50-150 |
| 6 | Phytosterol* | - | - | - | - | Coco Glucosides | 65-140 |

| | | | | | | | |
|---|---|---|---|---|---|---|---|
| *) 58,1 Gew.-% β-Sitosterol, 29,8 Gew.-% Campesterol, 4,5 Gew.-% Stigmasterol; 3,8 Gew.-% Tocopherol; 0,4 Gew.-% Cholesterol; 0,3 Gew.-% Squalan; ad 100 Unverseifbares. | | | | | | | |

Die nachfolgende Tabelle 2 enthält eine Reihe von Formulierungsbeispielen mit Phytosterol-Nanopartikeln.

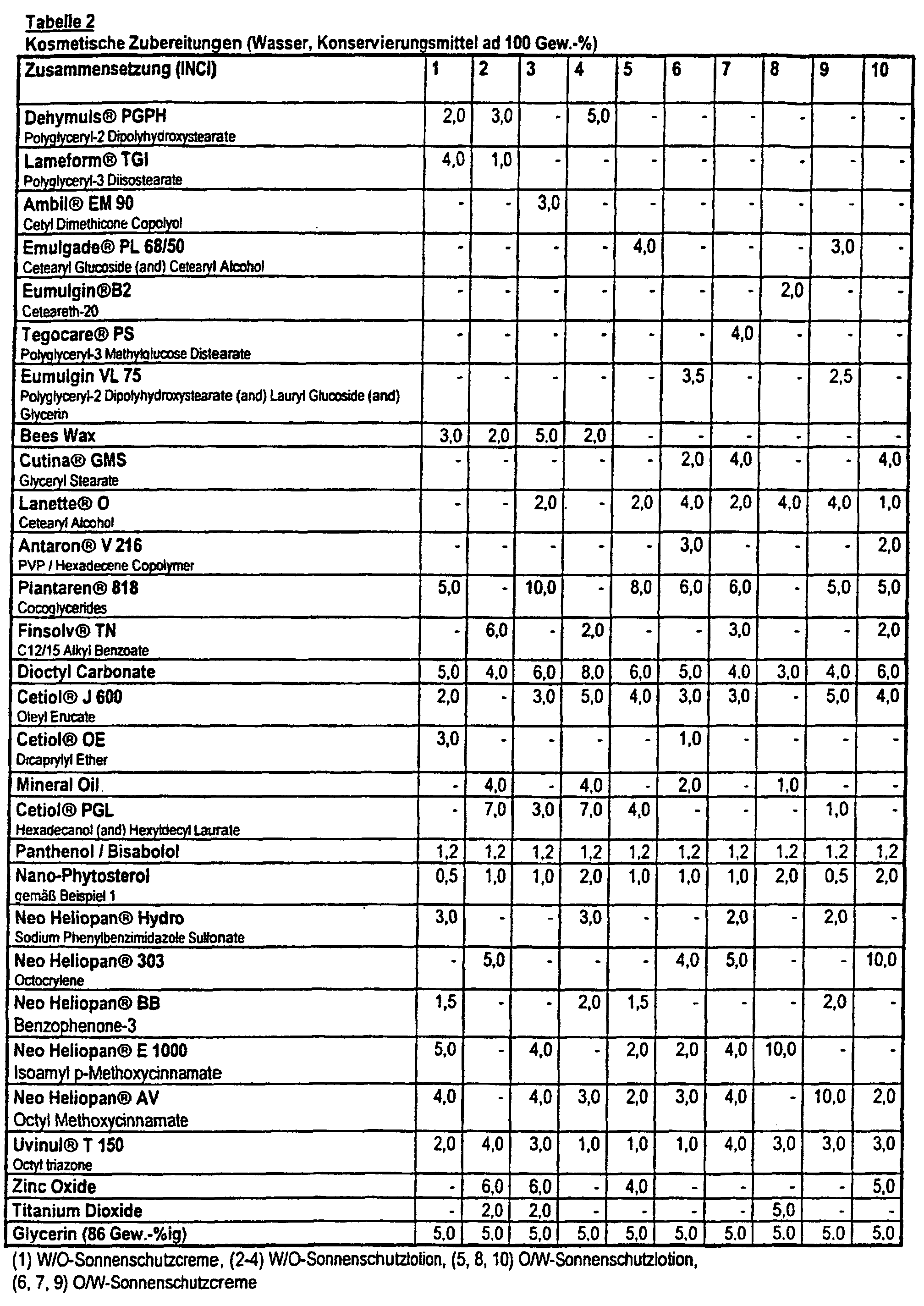

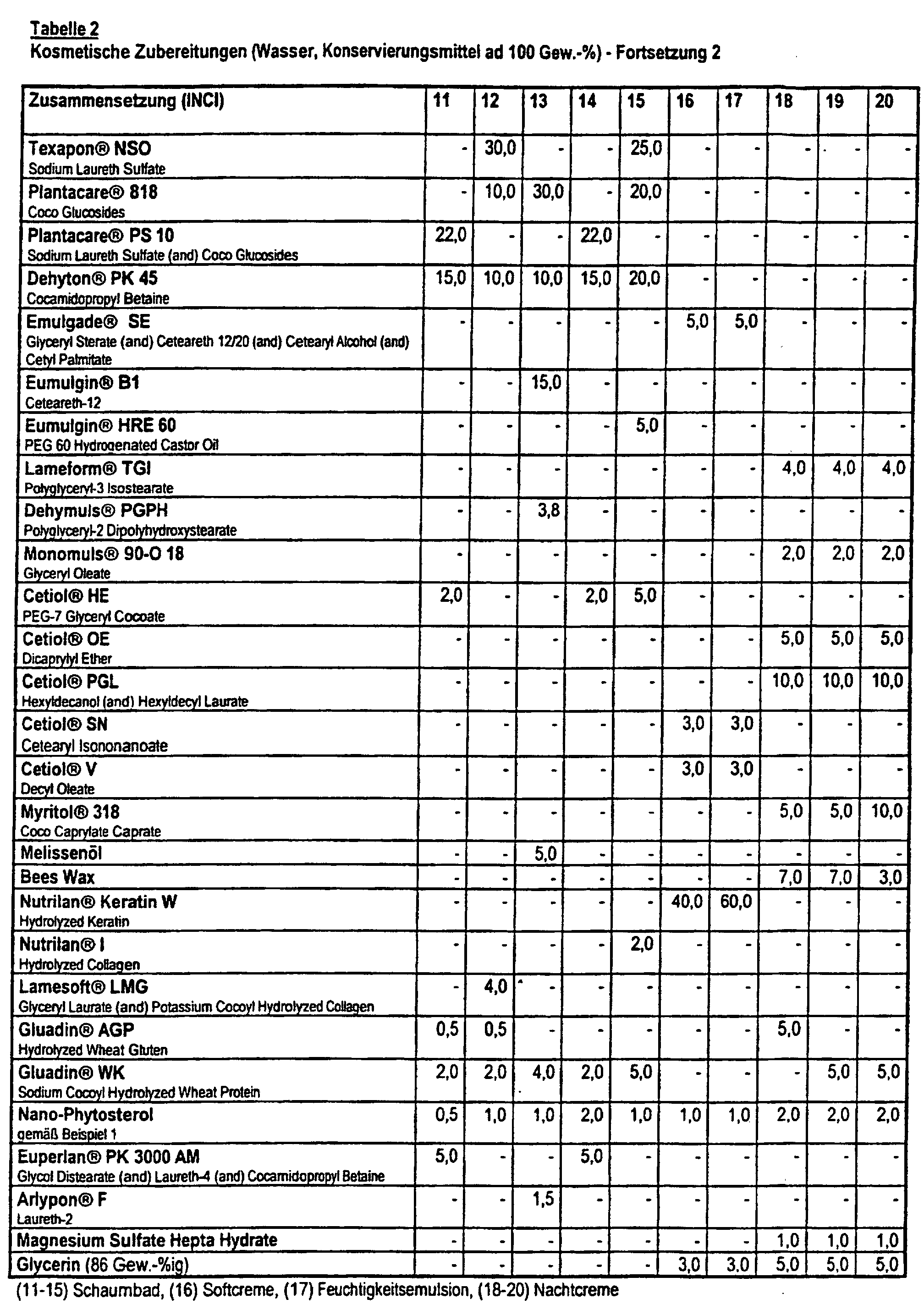

## Patentansprüche

1. Verwendung von nanoskaligen Sterolen und/oder Sterolestern mit Teilchendurchmessern im Bereich von 10 bis 300 nm zur Herstellung von kosmetischen und/oder pharmazeutischen Zubereitungen.

2. Verwendung nach Anspruch 1, **dadurch gekennzeichnet, daß** man Phytosterole oder deren Ester einsetzt.

3. Verwendung nach den Ansprüchen 1 und 2, **dadurch gekennzeichnet, daß** man Sitosterole oder deren Ester einsetzt.

4. Verwendung nach mindestens einem der Ansprüche 1 bis 3, **dadurch gekennzeichnet, daß** man nanoskalige Sterole und/oder Sterolester einsetzt, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

5. Verwendung nach mindestens einem der Ansprüche 1 bis 4, **dadurch gekennzeichnet, daß** man Nanopartikel einsetzt, welche von einem Schutzkolloid ummantelt vorliegen.

6. Verwendung nach Anspruch 5, **dadurch gekennzeichnet, daß** man als Schutzkolloid Polyvinyl-alkohol oder Polyethylenglycol einsetzt.

7. Verwendung nach mindestens einem der Ansprüche 1 bis 6, **dadurch gekennzeichnet, daß** man die Sterole und/oder Sterolester in Mengen von 0,1 bis 5 Gew.-% - bezogen auf die Zubereitungen - einsetzt.

8. Verwendung nach mindestens einem der Ansprüche 1 bis 7, **dadurch gekennzeichnet, daß** man die Sterole und/oder Sterolester zur Herstellung von Haarpflegemitteln einsetzt.

9. Verwendung nach mindestens einem der Ansprüche 1 bis 7, **dadurch gekennzeichnet, daß** man die Sterole und/oder Sterolester zur Herstellung von Hautpflegemitteln einsetzt.

10. Verwendung nach mindestens einem der Ansprüche 1 bis 7, **dadurch gekennzeichnet, daß** man die Sterole und/oder Sterolester zur Herstellung von Sonnenschutzmitteln einsetzt.

## Claims

1. The use of nanoscale sterols and/or sterol esters with particle diameters in the range from 10 to 300 nm for the production of cosmetic and/or pharmaceutical preparations.

2. The use claimed in claim 1, **characterized in that** phytosterols or their esters are used.

3. The use claimed in claims 1 and 2, **characterized in that** sitosterols or their esters are used.

4. The use claimed in at least one of claims 1 to 3, **characterized in that** nanoscale sterols and/or sterol esters obtained by
(a) dissolving the starting materials in a suitable solvent under supercritical or near-critical conditions,
(b) expanding the fluid mixture through a nozzle into a vacuum, a gas or a liquid and
(c) simultaneously evaporating the solvent.

5. The use claimed in at least one of claims 1 to 4, **characterized in that** nanoparticles coated with a protective colloid are used.

6. The use claimed in claim 5, **characterized in that** polyvinyl alcohol or polyethylene glycol is used as the protective colloid.

7. The use claimed in at least one of claims 1 to 6, **characterized in that** the sterols and/or sterol esters are used in quantities of 0.1 to 5% by weight, based on the preparations.

8. The use claimed in at least one of claims 1 to 7, **characterized in that** the sterols and/or sterol esters are used for the production of hair-care preparations.

9. The use claimed in at least one of claims 1 to 7, **characterized in that** the sterols and/or sterol esters are used for the production of skin-care preparations.

10. The use claimed in at least one of claims 1 to 7, **characterized in that** the sterols and/or sterol esters are used for the production of sun protection preparations.

## Revendications

1. Utilisation de stérols et/ou d'esters de stérols à l'échelle nano ayant des diamètres de particules dans la zone de 10 à 300 nm pour la production de préparations cosmétiques et/ou pharmaceutiques.

2. Utilisation selon la revendication 1,
**caractérisée en ce qu'**
on met en oeuvre des phytostérols ou leurs esters.

3. Utilisation selon les revendications 1 et 2,
**caractérisée en ce qu'**
on met en oeuvre des sitostérols ou leurs esters.

4. Utilisation selon au moins l'une des revendications 1 à 3,
**caractérisée en ce qu'**
on met en oeuvre des stérols et/ou des esters de stérols à l'échelle du nano que l'on obtient dans un procédé selon lequel on:
a) dissout dans un solvant approprié les matières premières dans des conditions super critiques ou proches de l'état critique,
b) détend le mélange fluide par l'intermédiaire d'une buse dans un vide, un gaz ou un liquide, et
c) évapore pour cela en même temps le solvant.

5. Utilisation selon au moins une revendication 1 à 4,
**caractérisée en ce qu'**
on met en oeuvre des nano particules qui se présentent enrobées par un colloïde protecteur.

6. Utilisation selon la revendication 5,
**caractérisée en ce qu'**
on met en oeuvre comme colloïde protecteur de l'alcool polyvinylique ou du polyéthylène glycol.

7. Utilisation selon l'une quelconque des revendications 1 à 6,
**caractérisée en ce qu'**
on met en oeuvre les stérols et/ou les esters de stérol en quantités allant de 0,1 à 5 % en poids rapporté aux préparations.

8. Utilisation selon au moins l'une quelconque des revendications 1 à 7,
**caractérisée en ce qu'**
on met en oeuvre les stérols et/ou les esters de stérols pour la production de produits de soin pour les cheveux.

9. Utilisation selon au moins l'une quelconque des revendications 1 à 7,
**caractérisée en ce qu'**
on met en oeuvre les stérols et/ou les esters de stérol pour la production de produits de soin pour la peau.

10. Utilisation selon au moins l'une quelconque des revendications 1 à 7,
**caractérisée en ce que**
les stérols et/ou stéolesters sont utilisés dans la préparation d'agents de protection solaire.
